# EUROPEAN PATENT SPECIFICATION

(11) **EP 3 019 146 B1**
(45) Date of publication and mention of the grant of the patent: **22.08.2018**
(21) Application number: 14748305.1
(22) Date of filing: 08.07.2014
(51) Int. Cl.: A61Q 1/02, A61K 8/19, A61K 8/36

(54) **LONG-WEAR COSMETIC COMPOSITION**
AUF DER HAUT HALTBARE KOSMETISCHE ZUSAMMENSETZUNG
COMPOSITION COSMETIQUE LONGUE TENUE

(30) Priority: 09.07.2013 JP 2013143329
(43) Date of publication of application: 18.05.2016
(73) Proprietor: L'Oréal, 75008 Paris (FR)
(72) Inventor: NIIMI, Rui, Kawasaki-shi Kanagawa 213-0012 (JP); SHIMIZU, Momoko, Kawasaki-shi Kanagawa 213-0012 (JP); KAMIDOI, Yuka, Kawasaki-shi Kanagawa 213-0012 (JP)
(74) Representative: Casalonga
(86) International application number: PCT/JP2014/068652
(87) International publication number: WO 2015/005490

(56) References cited:
- EP-A2- 2 250 995
- WO-A1-2012/081133
- GB-A- 2 047 731
- US-A1- 2005 187 128
- US-A1- 2005 191 328
- US-A1- 2012 148 511

## Description

### TECHNICAL FIELD

The present invention relates to a cosmetic composition for the skin. Particularly, the present invention relates to a cosmetic composition having a long-wear property.

### BACKGROUND ART

Sebum secreted from the skin affords an emollient effect to the stratum corneum of the skin, prevents intrusion of toxic substances or bacteria from the outside, and controls the release of substances, such as water, out of the body. However, excess sebum secretion has a demerit in that it may cause the makeup to come off. It leads to some phenomena, e.g., a "shiny" or "drab" appearance of the skin, or "unevenness", "rumpling", or "disappearance" of the makeup itself, and the like, caused by chronological changes in the cosmetic film formed on the skin.

A variety of investigations have already been conducted from the viewpoint of improving the long-wear property of makeup. For example, should highly moisture-absorbent or oil-absorbent substances, such as porous silica, calcium carbonate, magnesium carbonate, and crystalline cellulose, be mixed into cosmetics, the moisture and sebum components on the skin become adsorbed, which leads to a shortage of skin emollient components and causes a dry skin feeling, and a feeling of skin tightness or skin itchiness. This phenomenon is most likely to occur with persons with dry skin and normal skin, in particular with persons living in an environment where sweat or sebum is secreted less (e.g., persons working in an office). When used on oily skin, these substances have demerits which are apt to present luster due to excess sebum or oily components contained in the cosmetics, thus giving rise to a "shiny" look in the makeup finish.

Under these circumstances, there is a demand for a suitable skin-friendly cosmetic composition which improves the long-wear property of makeup, and can overcome the demerits of the above prior substances.

In view of the above, among the problems to be solved by the present invention is to provide a cosmetic composition which does not obstruct the skin physiology, has a good feeling on the skin, and has a superior long-wear effect for makeup.

Some patent publications describe technology of sebum gelification with metal oxides. For example, JP-B-4961082 discloses a silicone surface treatment with zinc oxide. JP-A-2002-20652 discloses composite particles of and spherical particles coated by silicone-treated zinc oxide. JP-B-3822782 teaches a combination of zinc oxide and hydroxyapatite in a cosmetically acceptable base. JP-B-3702072 discloses a composite where microparticle zinc oxide adheres to a silica surface. JP-B-3073890 discloses a silicone surface treatment with zinc oxide. JP-A-2011-51913 teaches a combination of microparticle titanium dioxide, and at least one chosen from magnesium oxide, calcium oxide, magnesium hydroxide, or calcium hydroxide, and clay. JP-A-2007-277191 teaches that citric acid stabilizes zinc oxide in w/o emulsion cosmetics, though it is silent on the sebum solidification effect.

### DISCLOSURE OF INVENTION

One of the objectives of the present invention is to provide a cosmetic composition which can provide a long-lasting effect, in particular a long-lasting makeup effect.
The above objective of the present invention can be achieved by a cosmetic composition comprising:
(i) at least one oxide of an alkaline earth metal,
(ii) at least one metal soap, which is a metal salt of C₁₀₋₃₀ fatty acid, and
(iii) an aqueous phase.
The (i) oxide of an alkaline earth metal is magnesium oxide. The (ii) metal soap has a metal moiety selected from the group consisting of magnesium, and calcium. In one embodiment, the (ii) metal soap, preferably a metal salt may have a fatty acid moiety selected from the group consisting of lauric acid, myristic acid, palmitic acid, stearic acid, and arachidic acid.

In one embodiment, the (ii) metal soap may be selected from the group consisting of magnesium stearate, calcium stearate, magnesium laurate, and calcium laurate.

In one embodiment, the amount of the (i) oxide of an alkaline earth metal is from 0.01% to 10.0%, preferably from 0.03% to 5.0 %, and more preferably from 0.05% to 3.0 % by weight in relation to the total weight of the cosmetic composition.
In one embodiment, the amount of the (ii) metal soap is from 0.1% to 10.0%, preferably from 0.3% to 5.0%, and more preferably from 0.5% to 3.0% by weight in relation to the total weight of the cosmetic composition.
In one embodiment, the ratio of the (i) the oxide of an alkaline earth metal and the (ii) metal soap is from 1:99 to 90:10, preferably from 3:97 to 70:30, and more preferably from 10:90 to 60:40.
In one embodiment, the cosmetic composition according to the present invention may be an emulsion, lotion, gel, or cream.
In one embodiment, the cosmetic composition according to the present invention may be a liquid foundation.

In one embodiment, the present invention relates to use of a combination of (i) at least one oxide of an alkaline earth metal which is magnesium oxide, (ii) at least one metal soap, which is a magnesium or calcium salt of C10-C30 fatty acid, and (iii) an aqueous phase as an agent for a long-wear effect.
The present invention also relates to a cosmetic process comprising applying to keratinous materials comprising human skin, a cosmetic composition as described hereinbefore. The process is preferably for improving a long-wear effect, more preferably for improving a long-wear makeup effect.
The cosmetic composition according to the present invention provides an improved rapid sebum solidification and/or gelification effect. As a result, the cosmetic composition according to the present invention maintains a long-wear makeup effect.

### BEST MODE FOR CARRYING OUT THE INVENTION

After diligent research, the inventors have discovered that it is possible to provide a cosmetic composition which can provide a long-wear effect, especially long-wear makeup effect.

The cosmetic composition according to the present invention can provide sebum solidification effects. Therefore, for example, the cosmetic effect is maintained for a long time period.

In the present invention, "sebum solidification" refers to a status in which sebum has been turned into a solid or gel, which can be restated as "sebum gelification". The time to solidify and/or gelify may not be specifically limited, but it is preferable to achieve sebum solidification and/or gelification within 1 hour, preferably within 30 minutes, and more preferably within 20 minutes.

In one embodiment, the weight ratio of the (i) oxide of an alkaline earth metal and the (ii) metal soap may be from 1:99 to 90:10, preferably from 3:97 to 70:30, and more preferably from 10:90 to 60:40.

### (i) Oxide of an alkaline earth metal

The alkaline earth metal constituting the oxide of an alkaline earth metal is magnesium oxide.

The oxide of an alkaline earth metal can be surface treated in a conventional manner.

In one embodiment, the oxide of an alkaline earth metal is not surface treated.

In one embodiment, the oxide of an alkaline earth metal may be pre-coated with a coating material such as a silicone compound, a fatty acid, a metal soap, a fluorine-based compound, a silane-coupling agent, and the like. A silicone compound is preferable. The percentage of the coating material in relation to the oxide of an alkaline earth metal may be from 0.1% to 10.0%, preferably from 0.3% to 8.0%, and more preferably from 0.5% to 7.0%.

The oxide of an alkaline earth metal is not limited, but preferably has an average particle size from 0.1 µm to 50 µm, more preferably from 0.5 µm to 30 µm, and even more preferably from 1 µm to 10 µm.

In one embodiment, the amount of the (i) oxide of an alkaline earth metal may be from 0.01% to 10.0%, preferably from 0.03% to 5.0%, and more preferably from 0.05% to 3.0% by weight in relation to the total weight of the cosmetic composition.

### (ii) Metal soap

The cosmetic composition according to the present invention comprises at least one metal soap, which is a magnesium or calcium salt of C10-30 fatty acid.More preferable species is magnesium.

### - Fatty acid moiety

The C₁₀₋₃₀ fatty acids useful in the present invention are any cosmetically or dermatologically acceptable, and, in general, physiologically acceptable fatty acid. The C₁₀₋₃₀ fatty acid may be saturated or partially unsaturated, and may consist of a straight chain or a branched chain. Examples of fatty acids include capric acid, lauric acid, myristic acid, palmitic acid, stearic acid, arachidic acid, behenic acid, ligoceric acid, cerotic acid, montanic acid, melissic acid, sapienic acid, oleic acid, linoleic acid, arachidonic acid, eicosapentaenoic acid, erucic acid, and docosahexaenoic acid. In an embodiment of the present invention, the fatty acid may be a C₁₂₋₂₀ fatty acid, and preferably C₁₂₋₁₈ fatty acid. In a particular embodiment of the present invention, the fatty acid may be lauric acid or stearic acid.

C₁₂₋₂₀ fatty acid may optionally be substituted by hydroxyl group. C₁₂₋₂₀ fatty acid includes a substituted C₁₂₋₂₀ fatty acid, such as a hydroxyl C₁₂₋₂₀ fatty acid. Hydroxyl C₁₂₋₂₀ fatty acid includes, for example, 2-Hydroxylaulic acid, 2-Hydroxymyristic acid, 2-Hydroxypalmitic acid, 2-Hydroxystearic acid, 2-Hydroxyarachidic acid, 3-Hydroxylaulic acid, 3-Hydroxytridecylic acid, 3-Hydroxymyristic acid, 3-Hydroxypalmitic acid, 3-Hydroxymargalic acid, 3-Hydroxystearic acid, 6-Hydroxystearic acid, 12-Hydroxystearic acid, 15-Hydroxypentadecylic acid, 16-Hydroxypalmitic acid, 17-Hydroxymargalic acid, 20-Hydroxyarachidic acid.

Thus, the preferable metal soap contained in the cosmetic composition according to the present invention may be magnesium stearate, calcium stearate, magnesium laurate, calcium laurate, and the like.

The (ii) metal soap used in the present invention is not a part of a coating material of substrates such as a pigment or filler, and is not complexed with substrates such as a pigment or filler.

The metal soap is not limited, but preferably it may have an average particle size from 0.1 µm to 50 µm, preferably from 0.5 µm to 30 µm, and more preferably from 1 µm to 10 µm.

In one embodiment, the amount of the (ii) metal soap may be from 0.1% to 10.0%, preferably from 0.3% to 5.0%, and more preferably from 0.5% to 3.0% by weight in relation to the total weight of the cosmetic composition.

### Physiologically acceptable medium

Besides the compounds indicated previously, the cosmetic composition according to the present invention comprises a physiologically acceptable medium.

The term "physiologically acceptable medium" is intended to denote a medium that is particularly suitable for applying a composition according to the present invention to the skin.

The physiologically acceptable medium is generally adapted to the nature of the support onto which the cosmetic composition is to be applied, and also to the form in which the cosmetic composition is to be packaged.

The cosmetic composition according to the present invention may be a dispersion or an emulsion. A dispersion may be made as an aqueous phase or as an oily phase. An emulsion may have an oily or aqueous continuous phase. Such an emulsion may be, for example, an inverse (W/O) emulsion or a direct (O/W) emulsion, or alternatively a multiple emulsion (W/O/W or O/W/O).

### (c) Aqueous phase

The cosmetic composition according to the present invention may advantageously comprise an aqueous phase. The aqueous phase comprises water. The water that is suitable for use in the present invention may be a floral water such as cornflower water and/or a mineral water such as Vittel water, Lucas water, or La Roche Posay water, and/or a spring water.
The aqueous phase may also comprise water-miscible organic solvents (at room temperature: 25°C), for instance monoalcohols containing from 2 to 6 carbon atoms, such as ethanol or isopropanol; polyols especially containing from 2 to 20 carbon atoms, preferably containing from 2 to 10 carbon atoms, and preferentially containing from 2 to 6 carbon atoms, such as glycerol, propylene glycol, butylene glycol, pentylene glycol, hexylene glycol, dipropylene glycol, or diethylene glycol; glycol ethers (especially containing from 3 to 16 carbon atoms) such as mono-, di-, or tripropylene glycol (C₁-C₄)alkyl ethers, mono-, di-, or triethylene glycol (C₁-C₄)alkyl ethers, and mixtures thereof.

The aqueous phase may also comprise stabilizers, for example sodium chloride, magnesium dichloride or magnesium sulfate.

The aqueous phase may also comprise any water-soluble or water-dispersible compound that is compatible with an aqueous phase, such as gelling agents, film-forming polymers, thickeners, or surfactants, and mixtures thereof.

In particular, the cosmetic composition according to the present invention may comprise an aqueous phase in a content ranging from 1% to 80% by weight, especially from 5% to 50%, and more particularly from 10% to 45% by weight relative to the total weight of the cosmetic composition.

### Fatty phase

The cosmetic composition according to the present invention may comprise at least one liquid and/or solid fatty phase.

According to one embodiment, the cosmetic composition according to the present invention is in the form of an emulsion.

In particular, the cosmetic composition according to the present invention may comprise at least one liquid fatty phase, especially at least one oil as mentioned below.

The term "oil" means any fatty substance that is in liquid form at room temperature (20-25°C) and at atmospheric pressure.

The composition of the present invention may comprise a liquid fatty phase in a content ranging from 1% to 90%, in particular from 5% to 80%, in particular from 10% to 70%, and more particularly from 20% to 50% by weight relative to the total weight of the cosmetic composition.

The oily phase that is suitable for preparing the cosmetic compositions according to the present invention may comprise hydrocarbon-based oils, silicone oils, fluoro oils, or non-fluoro oils, or mixtures thereof.

The oils may be volatile or non-volatile. The oils may be of animal, plant, mineral, or synthetic origin. The term "non-volatile oil" means an oil that remains on the skin or the keratin fiber at room temperature and atmospheric pressure. More specifically, a non-volatile oil has an evaporation rate strictly less than 0.01 mg/cm²/min.

To measure this evaporation rate, 15 g of oil or of an oil mixture to be tested are placed in a crystallizing dish 7 cm in diameter, which is placed on a balance in a large chamber of about 0.3 m³ that is temperature-regulated, at a temperature of 25°C, and hygrometry-regulated, at a relative humidity of 50%. The liquid is allowed to evaporate freely, without stirring it, while providing ventilation by means of a fan (Papst-Motoren, reference 8550 N, rotating at 2700 rpm) placed in a vertical position above the crystallizing dish containing said oil or said mixture, the blades being directed towards the crystallizing dish, 20 cm away from the bottom of the crystallizing dish. The mass of oil remaining in the crystallizing dish is measured at regular intervals. The evaporation rates are expressed in mg of oil evaporated per unit of area (cm²) and per unit of time (minutes).

The term "volatile oil" means any non-aqueous medium that is capable of evaporating on contact with the skin or the lips in less than one hour, at room temperature and atmospheric pressure. The volatile oil is a cosmetic volatile oil, which is liquid at room temperature. More specifically, a volatile oil has an evaporation rate of between 0.01 and 200 mg/cm²/min, limits included.

For the purposes of the present invention, the term "silicone oil" means an oil comprising at least one silicon atom, and especially at least one Si-O group.

The term "fluoro oil" means an oil comprising at least one fluorine atom.

The term "hydrocarbon-based oil" means an oil mainly containing hydrogen and carbon atoms.

The oils may optionally comprise oxygen, nitrogen, sulfur, and/or phosphorus atoms, for example in the form of hydroxyl or acid radicals.

### Volatile oils

The volatile oils may be chosen from hydrocarbon-based oils containing from 8 to 16 carbon atoms, and especially C₈-C₁₆ branched alkanes (also known as isoparaffins), for instance isododecane (also known as 2,2,4,4,6-pentamethylheptane), isodecane, and isohexadecane, for instance the oils sold under the trade names Isopar® or Permethyl®.

Volatile oils that may also be used include volatile silicones, for instance volatile linear or cyclic silicone oils, especially those with a viscosity of less than or equal to 8 centistokes (cSt) (8 × 10⁻⁶ m²/s), and especially containing from 2 to 10 silicon atoms and in particular from 2 to 7 silicon atoms, these silicones optionally comprising alkyl or alkoxy groups containing from 1 to 10 carbon atoms. As volatile silicone oils that may be used in the present invention, mention may be made especially of dimethicones with viscosities of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyl-tetrasiloxane, and dodecamethylpentasiloxane, and mixtures thereof.

According to one embodiment, a composition of the present invention may comprise from 1% to 80% by weight, or even from 5% to 70% by weight, or even from 10% to 60% by weight, and especially from 15% to 50% by weight of volatile oil relative to the total weight of the cosmetic composition.

### Non-volatile oils

The non-volatile oils may be chosen especially from non-volatile hydrocarbon-based, fluoro and/or silicone oils.

Non-volatile hydrocarbon-based oils that may especially be mentioned include:
- hydrocarbon-based oils of animal origin, such as perhydrosqualene,
- hydrocarbon-based oils of plant origin, such as phytostearyl esters, such as phytostearyl oleate, phytostearyl isostearate, and lauroyl/octyldodecyl/phytostearyl glutamate (Ajinomoto, Eldew PS203), triglycerides formed from fatty acid esters of glycerol, in particular in which the fatty acids may have chain lengths ranging from C₄ to C₃₆, and especially from C₁₈ to C₃₆, these oils possibly being linear or branched, and saturated or unsaturated; these oils may especially be heptanoic or octanoic triglycerides, shea oil, alfalfa oil, poppy oil, winter squash oil, millet oil, barley oil, quinoa oil, rye oil, candlenut oil, passionflower oil, shea butter, aloe vera oil, sweet almond oil, peach stone oil, groundnut oil, argan oil, avocado oil, baobab oil, borage oil, broccoli oil, calendula oil, camelina oil, canola oil, carrot oil, safflower oil, flax oil, rapeseed oil, cotton oil, coconut oil, marrow seed oil, wheatgerm oil, jojoba oil, lily oil, macadamia oil, corn oil, meadowfoam oil, St John's Wort oil, monoi oil, hazelnut oil, apricot kernel oil, walnut oil, olive oil, evening primrose oil, palm oil, blackcurrant pip oil, kiwi seed oil, grapeseed oil, pistachio oil, winter squash oil, pumpkin oil, musk rose oil, sesame oil, soybean oil, sunflower oil, castor oil, and watermelon seed oil, and mixtures thereof, or alternatively caprylic/capric acid triglycerides, such as those sold by the company Stearineries Dubois or those sold under the names Miglyol 810®, 812®, and 818® by the company Dynamit Nobel,
- linear or branched hydrocarbons of mineral or synthetic origin, such as liquid paraffins and derivatives thereof, petroleum jelly, polydecenes, polybutenes, hydrogenated polyisobutene such as Parleam, and squalane;
- synthetic ethers containing from 10 to 40 carbon atoms;
- synthetic esters, for instance the oils of formula R₁COOR₂, in which R₁ represents a linear or branched fatty acid residue containing from 1 to 40 carbon atoms and R₂ represents a hydrocarbon-based chain, which is especially branched, containing from 1 to 40 carbon atoms, on condition that the sum of the number of carbon atoms in the chains R₁ and R₂ is greater than or equal to 10. The esters may be chosen especially from fatty acid esters of alcohols, for instance cetostearyl octanoate, isopropyl alcohol esters, such as isopropyl myristate, isopropyl palmitate, ethyl palmitate, 2-ethylhexyl palmitate, isopropyl stearate, isopropyl isostearate, isostearyl isostearate, and octyl stearate, hydroxylated esters, for instance isostearyl lactate, octyl hydroxystearate, diisopropyl adipate, heptanoates, and especially isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate, and 2-ethylhexyl palmitate, alkyl benzoates, polyethylene glycol diheptanoate, propylene glycol 2-diethylhexanoate, and mixtures thereof, C₁₂-C₁₅ alcohol benzoates, hexyl laurate, neopentanoic acid esters, for instance isodecyl neopentanoate, isotridecyl neopentanoate, isostearyl neopentanoate, and octyldodecyl neopentanoate, isononanoic acid esters, for instance isononyl isononanoate, isotridecyl isononanoate, and octyl isononanoate, hydroxylated esters, for instance isostearyl lactate and diisostearyl malate,
- polyol esters and pentaerythritol esters, for instance dipentaerythrityl tetrahydroxystearate/tetraisostearate,
- esters of diol dimers and of diacid dimers, such as Lusplan DD-DA5® and Lusplan DD-DA7® sold by the company Nippon Fine Chemical and described in patent application US 2004-175 338,
- copolymers of a diol dimer and of a diacid dimer and esters thereof, such as dilinoleyl diol dimer/dilinoleic dimer copolymers, and esters thereof, for instance Plandool-G,
- copolymers of polyols and of diacid dimers, and esters thereof, such as Hailuscent ISDA or dilinoleic acid/butanediol copolymers,
- fatty alcohols that are liquid at room temperature, with a branched and/or unsaturated carbon-based chain containing from 12 to 26 carbon atoms, for instance 2-octyldodecanol, isostearyl alcohol, oleyl alcohol, 2-hexyldecanol, 2-butyloctanol, and 2-undecyl-pentadecanol;
- C₁₂-C₂₂ higher fatty acids, such as oleic acid, linoleic acid, or linolenic acid, and mixtures thereof,
- dialkyl carbonates, the two alkyl chains possibly being identical or different, such as dicaprylyl carbonate sold under the name Cetiol CC® by Cognis,
- oils of high molar mass, in particular having a molar mass ranging from about 400 to about 10,000 g/mol, in particular from about 650 to about 10 000 g/mol, more particularly from about 750 to about 7500 g/mol, and even more particularly ranging from about 1000 to about 5000 g/mol. As oils of high molar mass that may be used in the present invention, mention may especially be made of oils chosen from:
   lipophilic polymers,
   linear fatty acid esters with a total carbon number ranging from 35 to 70,
   hydroxylated esters,
   aromatic esters,
   C₂₄-C₂₈ branched fatty acid or fatty alcohol esters,
   silicone oils,
   oils of plant origin, and
   mixtures thereof;
- optionally partially hydrocarbon-based and/or silicone fluoro oils, for instance fluorosilicone oils, fluoropolyethers, and fluorosilicones as described in document EP-A-847 752;
- silicone oils, for instance linear or cyclic non-volatile polydimethylsiloxanes (PDMS); polydimethylsiloxanes comprising alkyl, alkoxy, or phenyl groups, which are pendant or at the end of a silicone chain, these groups containing from 2 to 24 carbon atoms; phenyl silicones, for instance phenyl trimethicones, phenyl dimethicones, phenyl trimethylsiloxy diphenyl siloxanes, diphenyl dimethicones, diphenyl methyldiphenyl trisiloxanes, and 2-phenylethyl trimethylsiloxy silicates, and
- mixtures thereof.

According to one particular embodiment, the fatty phase of the cosmetic composition according to the present invention may contain only volatile compounds.

### Dyestuffs

The cosmetic composition according to the present invention may also comprise at least one dyestuff.

The amount of dyestuff(s) in the cosmetic composition according to the present invention will generally range from 0 to 25%, preferably from 2 to 15%, and more preferably from 5 to 15% by weight of the total weight of the cosmetic composition.

The cosmetic composition according to the present invention may incorporate at least one dyestuff chosen from mineral or organic pigments conventionally used in cosmetic compositions, liposoluble or water-soluble dyes, materials with a specific optical effect, and mixtures thereof.

The term "pigments" should be understood to mean white or colored, inorganic or organic particles which are insoluble in an aqueous solution and are intended for coloring and/or opacifying the resulting film.

As inorganic pigments that can be used in the present invention, mention may be made of titanium oxides, zirconium oxides, or cerium oxides, and also zinc oxides, iron oxides or chromium oxides, ferric blue, manganese violet, ultramarine blue and chromium hydrate. According to one particular mode of the present invention, the mineral pigments will be chosen from iron oxides and titanium oxides, and mixtures thereof.

It may also be a pigment having a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

The colorant may also comprise a pigment having a structure which may be, for example, of the type such as silica microspheres containing iron oxide. An example of a pigment having this structure is the product sold by the company Miyoshi under the reference PC Ball PC-LL-100 P, this pigment being constituted of silica microspheres containing yellow iron oxide.

Among the organic pigments that may be used in the present invention, mention may be made of carbon black, pigments of D&C type, lakes based on cochineal carmine or on barium, strontium, calcium, or aluminium, or alternatively the diketopyrrolopyrroles (DPP) described in documents EP 0 542 669, EP 0 787 730, EP 0 787 731, and WO 96/08537.

The cosmetic composition according to the present invention may also comprise water-soluble or fat-soluble dyes. The liposoluble dyes are, for example, Sudan Red, DC Red 17, DC Green 6, β -carotene, soybean oil, Sudan Brown, DC Yellow 11, DC Violet 2, DC Orange 5, and quinoline yellow. The water-soluble dyes are, for example, beetroot juice and caramel.

### Additional fillers

The cosmetic composition according to the present invention may also comprise at least one additional filler, of organic or mineral nature, making it possible especially to give it additional matte-effect or covering properties, and/or improved stability with regard to exudation and migration-resistance properties after application.

The term "filler" should be understood to mean colorless or white solid particles of any shape which are in a form that is insoluble and dispersed in the medium of the cosmetic composition. These particles, of mineral or organic nature, can give body or rigidity to the cosmetic composition and/or softness and uniformity to the makeup.

The fillers used in the cosmetic compositions according to the present invention may be in lamellar, globular, or spherical form, in the form of fibers, or in any other intermediate form between these defined forms.

The fillers according to the present invention may or may not be surface-coated, and in particular they may be surface-treated with silicones, amino acids, fluoro derivatives, or any other substance that promotes the dispersion and compatibility of the filler in the cosmetic composition.

Examples of mineral fillers that may be mentioned include talc, mica, silica, kaolin, calcium carbonate, magnesium carbonate, hydroxyapatite, glass, or ceramic microcapsules.

Examples of organic fillers that may be mentioned include polyethylene powder or polymethyl methacrylate powder, polytetrafluoroethylene (Teflon) powders, lauroyl lysine, hexamethylene diisocyanate/trimethylol hexyl lactone copolymer powder (Plastic Powder from Toshiki), silicone resin microbeads (for example Tospearl from Toshiba), natural or synthetic micronized waxes, metal soaps derived from organic carboxylic acids containing from 8 to 22 carbon atoms and preferably from 12 to 18 carbon atoms, for example zinc stearate, magnesium stearate, lithium stearate, zinc laurate, or magnesium myristate, and polyurethane powders, in particular crosslinked polyurethane powders comprising a copolymer, the said copolymer comprising trimethylol hexyl lactone. It may in particular be a hexamethylene diisocyanate/trimethylol hexyl lactone polymer. Such particles are especially commercially available, for example, under the name Plastic Powder D-400® or Plastic Powder D-800® from the company Toshiki, and mixtures thereof.

The amount of filler(s) in the cosmetic composition of the present invention will generally range from 0 to 25%, preferably from 2 to 15%, and more preferably from 5 to 15% by weight of the total weight of the cosmetic composition.

### Additives

In a particular embodiment, the cosmetic composition according to the present invention further comprises at least one compound chosen from water, hydrophilic solvents, lipophilic solvents, oils, and mixtures thereof.

The cosmetic composition according to the present invention may also comprise any additive usually used in the field under consideration, chosen, for example, from gums, anionic, cationic, amphoteric, or nonionic surfactants, silicone surfactants, resins, thickening agents, structuring agents such as waxes, dispersants, antioxidants, essential oils, preserving agents, fragrances, neutralizers, antiseptics, UV-screening agents, cosmetic active agents, such as vitamins, moisturizers, emollients, or collagen-protecting agents, and mixtures thereof.

It is a matter of routine operations for a person skilled in the art to adjust the nature and amount of the additives present in the cosmetic compositions in accordance with the present invention such that the desired cosmetic properties and stability properties thereof are not thereby affected.

The cosmetic composition according to the present invention may be in the form of a skin makeup product, in particular a foundation, a hot-cast foundation product, a body makeup product, a concealer, an eyeshadow, a lipstick, or a body deodorant. The cosmetic composition may be in the form of a gel, in the form of cream; in the form of a stick or wand, or in the form of a soft paste. In a particular embodiment, the cosmetic composition may be a liquid foundation.

A care composition according to the present invention may in particular be an anti-sun composition. Preferably, the cosmetic composition according to the present invention is in the form of a fluid primer or a fluid foundation.

In one embodiment, the cosmetic composition may be in the form of emulsion or in the form of a clear lotion.

In a particular embodiment, the present invention relates to use of a cosmetic composition comprising (i) at least one oxide of an alkaline earth metal and (ii) at least one metal soap, preferably a metal salt of C₁₀₋₃₀ fatty acid as an agent for a long-wear makeup effect.

### [Cosmetic process]

The present invention also concerns a cosmetic process comprising a step of applying the cosmetic composition according to the present invention, on the skin, in particular the face. In a particular embodiment, the cosmetic composition is applied alone or as a base or primer under a skin care product or a makeup product. The cosmetic process preferably includes making up and/or caring for the skin, preferably facial skin.

The cosmetic composition used in the cosmetic process according to the present invention is preferably of the leave-in type. The term "leave-in" means a composition that is not intended to be washed out or removed immediately after application.

The cosmetic process according to the present invention can provide sebum solidification or gelification effects, as well as a long-wear effect without causing shine. Thus, the cosmetic process according to the present invention can provide a long-wear effect on the skin over time even under hot and/or humid conditions, for example, during the summer.

### EXAMPLES

The present invention will be described in a more detailed manner by way of examples. However, these examples should not be construed as limiting the scope of the present invention.

### Test Example 1 and Test Comparative Examples 1 to 3

### [Preparation]

The following compositions according to Example 1 (Ex. 1) and Comparative Examples 1 to 3 (Comp. Ex. 1 to 3) were respectively prepared by mixing the components shown in Table 1. The numerical values for the amounts of the components are all based on "% by weight" as active raw materials.

### Sebum gelification test

### [Protocol]

The gelification speed of artificial sebum was measured by using the following combination.

The mixtures below were each stirred at room temperature for 10 min by a magnetic stirrer. When the magnetic stirrer was stopped due to sebum gelification (sodification) or the composition was moved to the flask wall by the magnetic stirrer and was not returned to the magnetic stirrer, this moment was determined to be the gelification time. One hour after the start of stirring, the cosmetic composition was applied over a contrast card with a 30 µm applicator. The surface shine was then measured at a 60° gloss value of a Glossmeter.

**Table 1: Composition and results of sebum gelification test**

| Composition (g) | Ex. 1 | Ex. 1' | Comp. Ex. 1 | Comp. Ex. 2 | Comp. Ex. 3 |
|---|---|---|---|---|---|
| Magnesium oxide (Magnesium oxide light: Dr. Paul Lohmann) | 0.1 | 0.1 | 0.1 | - | - |
| Magnesium stearate (Stearinerie Dubois) | 0.1 | - | - | 0.1 | - |
| Magnesium 12-hydroxystearate | - | 0.1 | - | - | - |
| Water | 0.25 | 0.25 | 0.25 | 0.25 | 0.25 |
| Artificial Sebum (see table 2) | 5 | 5 | 5 | 5 | 5 |
| Gelification time | 20 min | 6 min | No gel | No gel | No gel |
| 60° Gloss (30 um application on contrast card) | 6.3 | 5.1 | 9.6 | 75 | 84.1 |

Table 2 shows the composition of the artificial sebum used.

**Table 2: Composition of artificial sebum**

| **Ingredient** | Wt% |
|---|---|
| Triisostearin | 28.7 |
| Parleam | 13.7 |
| Oleic acid | 28.0 |
| Oleyl erucate | 22.9 |
| Octyldodecanol | 6.7 |
| **Total** | 100 |

### [Results]

As shown in Ex. 1 and Ex. 1', when magnesium oxide was mixed with magnesium stearate or magnesium 12-hydroxystearate and artificial sebum, the composition formed a gel. The gelified artificial sebum showed the lowest gloss. On the other hand, magnesium oxide alone (Comp. Ex. 1) or magnesium stearate alone (Comp. Ex. 2), did not form a gel. For reference, artificial sebum itself did not turn into a gel (Comp. Ex. 3).

Consequently, the combination of an oxide of a divalent metal (e.g., magnesium oxide) and a metal soap (e.g., magnesium stearate or magnesium 12-hydroxystearate) accelerated sebum gelification, and decreased the shine of sebum, which means a high matte effect.

Consequently, it was confirmed that a combination of magnesium oxide and magnesium stearate or magnesium 12-hydroxystearate showed gelification of artificial sebum and reduced shine of artificial sebum.

### Test Example 2

The weight ratio of magnesium oxide and magnesium stearate was evaluated by the sebum gelification test as shown above. A mixture of magnesium oxide and/or magnesium stearate according to the following weight ratio (0.1 g in total) was mixed with 10% of water and 2.0 g of artificial sebum as shown in Table 2. The compositions which failed to gelify in 10 minutes were judged as "NG (no good)".

**Table 3: Composition of magnesium oxide and/or magnesium stearate and results**

| Particle (weight ratio) | | Gelification time |
|---|---|---|
| Magnesium oxide (Magnesium oxide light: Dr. Paul Lohmann)/ magnesium stearate (Stearinerie Dubois) | 0 / 100 | NG |
| | 3 / 97 | 4 min |
| | 10 / 90 | 4 min |
| | 25 / 75 | 5 min |
| | 50 / 50 | 6 min |
| | 75 / 25 | 10 min |
| | 90 / 10 | 10 min |
| | 100 / 0 | NG |

### Formulation Example (Liquid Foundation)

### [Preparation]

In Table 4 below, all compositions are expressed in % by weight. They were prepared according to the same protocol as above. The oil phase was well mixed at room temperature, and the water phase was also mixed at room temperature. The oil phase and water phase were then emulsified at room temperature.

**Table 4: Liquid foundation formula**

| Formulation Example | Ex. 1 | Comp. Ex. 1 | Comp. Ex. 2 | Comp. Ex. 3 |
|---|---|---|---|---|
| PEG-10 Dimethicone (KF6017: Shinetsu) | 2.5 | 2.5 | 2.5 | 2.5 |
| Dimethicone (KF96L 2CS: Shinetsu) | 12.1 | 14.1 | 13.1 | 13.1 |
| Ethylhexyl Methoxycinnamate | 3 | 3 | 3 | 3 |
| Talc | 2.5 | 2.5 | 2.5 | 2.5 |
| Iron oxides | 11.9 | 11.9 | 11.9 | 11.9 |
| Magnesium Oxide (Magnesium oxide light: Dr. Paul Lohmann) | 1 | | 1 | |
| Magnesium Stearate (Stearinerie Dubois) | 1 | | | 1 |
| Water | qs.100 | qs.100 | qs.100 | qs.100 |
| Glycerin | 3.5 | 3.5 | 3.5 | 3.5 |
| Alcohol Denat. | 8 | 8 | 8 | 8 |
| Butylene Glycol | 3 | 3 | 3 | 3 |
| Total | 100 | 100 | 100 | 100 |

### Sebum resistance test of liquid foundation

### [Protocol]

Each of the liquid foundations (1.0 g) as prepared above and artificial sebum (0.2 g) were mixed, and the mixture was applied on artificial leather at 2.4 mg/cm², and then the artificial leather (SUPPLALE® from Idemitsu) was dried at 33°C for 30 min. The reflectance of the foundation film was measured by a goniophotometer (A). The reflectance of the foundation without artificial sebum was also measured (B).

### [Results]

Test results are shown in Table 5. The formula according to the present invention (Formulation Example 1) containing both magnesium oxide and magnesium stearate showed lower reflectance compared with comparative formulas (Formulation Comparative Examples 1, 2, and 3) in the presence of artificial sebum. This means that the formula according to the present invention has better sebum resistance than the comparative formula.

**Table 5: Results of sebum resistance test (Specular reflectance)**

| Formulation Example | Ex. 1 | Comp. Ex. 1 | Comp. Ex. 2 | Comp. Ex. 3 |
|---|---|---|---|---|
| With artificial sebum (A) | 90.4 | 191.3 | 151.8 | 145.6 |
| Without artificial sebum (B) | 34.1 | 46.5 | 36.6 | 49.4 |
| Difference: (A)-(B) | 56.3 | 144.8 | 115.2 | 96.2 |

### Formulation Example 2 (Water base foundation)

The composition of Table 6 was well mixed at room temperature. The obtained foundation had a long-wear effect.

**Table 6: Water base foundation formula**

| Formulation Example | Ex.2 |
|---|---|
| Magnesium Oxide (Magnesium oxide light: Dr. Paul Lohmann) | 1 |
| Magnesium Stearate | 2 |
| Iron Oxide | 11.9 |
| Glycerin | 7 |
| Alcohol Denat. | 10 |
| Butylene Glycol | 10 |
| Phenoxy Ethanol | 0.5 |
| Talc (Micro Ace P3: Nippon Talc) | 5 |
| Water | qs. 100 |
| Total | 100 |

### Test Example 3

Mixtures of magnesium oxide and each stearate metal soap were evaluated by the sebum gelification test as shown above. 0.1 g of magnesium oxide and 0.9 g of stearate metal soap below were mixed with 10% of water and 2.0 g of artificial sebum as shown in Table 2. Table 7 shows the time to gelify the artificial sebum.

**Table 7: Metal type and result of gelling time**

| Composition (MgO+stearate metal soap) | | Gelling time (with 10% water) |
|---|---|---|
| Magnesium oxide (Magnesium oxide light: Dr. Paul Lohmann) | Magnesium stearate (Stearinerie Dubois) | 2 min |
| | Calcium stearate (Stearinerie Dubois) | 10 min |
| | Zinc stearate (Peter Greven or NOF or Kawamura Kasei) | 18 min |

This table shows that magnesium stearate, calcium stearate, and zinc stearate gelified the artificial sebum in the presence of water.

## Claims

1. A cosmetic composition comprising
(i) at least one oxide of an alkaline earth metal,
(ii) at least one metal soap which is a magnesium or calcium salt of C₁₀-₃₀ fatty acid, and
(iii) an aqueous phase,
wherein the (i) oxide of an alkaline earth metal is magnesium oxide.

2. The cosmetic composition according to claim 1, wherein the (ii) metal soap has a fatty acid moiety selected from the group consisting of lauric acid, myristic acid, palmitic acid, stearic acid, and arachidic acid.

3. The cosmetic composition according to claim 1 or 2, wherein (ii) the metal soap is selected from the group consisting of magnesium stearate, calcium stearate, magnesium laurate, and calcium laurate.

4. The cosmetic composition according to any one of claims 1 to 3, wherein the amount of the (i) oxide of an alkaline earth metal is from 0.01% to 10.0%, preferably from 0.03% to 5.0 %, and more preferably from 0.05% to 3.0% by weight in relation to the total weight of the cosmetic composition.

5. The cosmetic composition according to any one of claims 1 to 4, wherein the amount of the (ii) metal soap is from 0.1% to 10.0%, preferably from 0.3% to 5.0%, and more preferably from 0.5% to 3.0% by weight in relation to the total weight of the cosmetic composition.

6. The cosmetic composition according to any one of claims 1 to 5, wherein the ratio of the (i) the oxide of an alkaline earth metal and the (ii) metal soap is from 1:99 to 90:10, preferably from 3:97 to 70:30, and more preferably from 10:90 to 60:40.

7. The cosmetic composition according to any one of claims 1 to 6, which is an emulsion, lotion, gel, or cream.

8. The cosmetic composition according to any one of claims 1 to 7, which is a liquid foundation.

9. Use of a combination of (i) at least one oxide of an alkaline earth metal which is magnesium oxide, and (ii) at least one metal soap which is a magnesium or calcium salt of C₁₀-₃₀ fatty acid in a composition comprising an aqueous phase as an agent for a long-wear effect.

10. A cosmetic process comprising applying to keratinous materials comprising human skin, a cosmetic composition according to any one of claims 1 to 8.

11. The cosmetic process according to claim 10, for improving a long-wear effect.

## Patentansprüche

1. Kosmetische Zusammensetzung, umfassend:
(i) wenigstens ein Oxid eines Erdalkalimetalls,
(ii) wenigstens eine Metallseife, bei der es sich um ein Magnesium- oder Calciumsalz einer C₁₀₋₃₀-Fettsäure handelt, und
(iii) eine wässrige Phase,
wobei das (i) Oxid eines Erdalkalimetalls Magnesiumoxid ist.

2. Kosmetische Zusammensetzung nach Anspruch 1, wobei die (ii) Metallseife eine Fettsäurekomponente aufweist, die ausgewählt ist aus der Gruppe bestehend aus Laurinsäure, Myristinsäure, Palmitinsäure, Stearinsäure und Arachinsäure.

3. Kosmetische Zusammensetzung nach Anspruch 1 oder 2, wobei (ii) die Metallseife ausgewählt ist aus der Gruppe bestehend aus Magnesiumstearat, Calciumstearat, Magnesiumlaurat und Calciumlaurat.

4. Kosmetische Zusammensetzung nach einem der Ansprüche 1 bis 3, wobei die Menge des (i) Oxids eines Erdalkalimetalls 0,01 Gew.-% bis 10,0 Gew.-%, vorzugsweise 0,03 Gew.-% bis 5,0 Gew.-% und insbesondere 0,05 Gew.-% bis 3,0 Gew.-%, bezogen auf das Gesamtgewicht der kosmetischen Zusammensetzung, beträgt.

5. Kosmetische Zusammensetzung nach einem der Ansprüche 1 bis 4, wobei die Menge der (ii) Metallseife 0,1 Gew.-% bis 10,0 Gew.-%, vorzugsweise 0,3 Gew.-% bis 5,0 Gew.-% und insbesondere 0,5 Gew.-% bis 3,0 Gew.-%, bezogen auf das Gesamtgewicht der kosmetischen Zusammensetzung, beträgt.

6. Kosmetische Zusammensetzung nach einem der Ansprüche 1 bis 5, wobei das Verhältnis des (i) Oxids eines Erdalkalimetalls zu der (ii) Metallseife 1:99 bis 90:10, vorzugsweise 3:97 bis 70:30 und insbesondere 10:90 bis 60:40 beträgt.

7. Kosmetische Zusammensetzung nach einem der Ansprüche 1 bis 6, bei der es sich um eine Emulsion, eine Lotion, ein Gel oder eine Creme handelt.

8. Kosmetische Zusammensetzung nach einem der Ansprüche 1 bis 7, bei der es sich um eine flüssige Grundierung handelt.

9. Benutzung einer Kombination aus (i) wenigstens einem Oxid eines Erdalkalimetalls, bei dem es sich um Magnesiumoxid handelt, und (ii) wenigstens einer Metallseife, bei der es sich um ein Magnesium- oder Calciumsalz einer C₁₀₋₃₀-Fettsäure handelt, in einer Zusammensetzung, die eine wässrige Phase umfasst, als ein Mittel für einen Effekt einer langen Haltbarkeit auf der Haut.

10. Kosmetisches Verfahren, das ein Aufbringen einer kosmetischen Zusammensetzung nach einem der Ansprüche 1 bis 8 auf Keratinmaterialien umfasst, die menschliche Haut umfassen.

11. Kosmetisches Verfahren nach Anspruch 10 zum Verbessern eines Effekts einer langen Haltbarkeit auf der Haut.

## Revendications

1. Composition cosmétique comprenant :
(i) au moins un oxyde d'un métal alcalino-terreux,
(ii) au moins un savon métallique qui est un sel de calcium ou de magnésium d'un acide gras en C₁₀-C₃₀, et
(iii) une phase aqueuse,
l'oxyde d'un métal alcalino-terreux (i) étant l'oxyde de magnésium.

2. Composition cosmétique selon la revendication 1, dans laquelle le savon métallique (ii) comporte un fragment acide gras choisi dans l'ensemble constitué par l'acide laurique, l'acide myristique, l'acide palmitique, l'acide stéarique et l'acide arachidique.

3. Composition cosmétique selon la revendication 1 ou 2, dans laquelle le savon métallique (ii) est choisi dans l'ensemble constitué par le stéarate de magnésium, le stéarate de calcium, le laurate de magnésium el le laurate de calcium.

4. Composition cosmétique selon l'une quelconque des revendications 1 à 3, dans laquelle la quantité de l'oxyde d'un métal alcalino-terreux (i) vaut de 0,01 % à 10,0 %, de préférence de 0,03 % à 5,0 %, et de façon plus particulièrement préférée de 0,05 % à 3,0 % en poids par rapport au poids total de la composition cosmétique.

5. Composition cosmétique selon l'une quelconque des revendications 1 à 4, dans laquelle la quantité du savon métallique (ii) vaut de 0,1 % à 10,0 %, de préférence de 0,3 % à 5,0 %, et de façon plus particulièrement préférée de 0,5 % à 3,0 % en poids par rapport au poids total de la composition cosmétique.

6. Composition cosmétique selon l'une quelconque des revendications 1 à 5, dans laquelle le rapport de l'oxyde d'un métal alcalino-terreux (i) au savon métallique (ii) vaut de 1:99 à 90:10, de préférence de 3:97 à 70:30, et de façon plus particulièrement préférée de 10:90 à 60:40.

7. Composition cosmétique selon l'une quelconque des revendications 1 à 6, qui est une émulsion, une lotion, un gel ou une crème.

8. Composition cosmétique selon l'une quelconque des revendications 1 à 7, qui est un fond de teint liquide.

9. Utilisation d'une association de (i) au moins un oxyde d'un métal alcalino-terreux qui est l'oxyde de magnésium et (ii) au moins un savon métallique qui est un sel de calcium ou de magnésium d'un acide gras en C₁₀-C₃₀, dans une composition comprenant une phase aqueuse, en tant qu'un agent pour un effet de longue tenue.

10. Procédé cosmétique comprenant l'application d'une composition cosmétique selon l'une quelconque des revendications 1 à 8 sur des matières kératiniques comprenant la peau humaine.

11. Procédé cosmétique selon la revendication 10, pour améliorer un effet de longue tenue.
